# EUROPEAN PATENT SPECIFICATION

(11) **EP 3 270 223 B1**
(45) Date of publication and mention of the grant of the patent: **05.04.2023**
(21) Application number: 16878084.9
(22) Date of filing: 13.10.2016
(51) Int. Cl.: H04N 9/31, G03B 21/20, G02B 27/01, G02B 26/10

(54) **PROJECTION APPARATUS AND METHOD**
PROJEKTIONSVORRICHTUNG UND -VERFAHREN
APPAREIL ET PROCÉDÉ DE PROJECTION

(30) Priority: 27.05.2016 CN 201610370520
(43) Date of publication of application: 17.01.2018
(73) Proprietor: Huawei Technologies Co., Ltd., Longgang District Shenzhen, Guangdong 518129 (CN)
(72) Inventor: LIN, Tao, Shenzhen Guangdong 518129 (CN)
(74) Representative: Epping - Hermann - Fischer
(86) International application number: PCT/CN2016/102008
(87) International publication number: WO 2017/201948

(56) References cited:
- WO-A1-00/62538
- WO-A1-2015/129584
- WO-A2-2007/116338
- CN-A- 101 023 387
- CN-A- 101 415 990
- CN-A- 104 076 507
- CN-A- 105 842 971
- CN-U- 201 974 578
- CN-U- 204 989 737
- JP-A- 2014 085 605
- US-A- 5 727 098
- US-A1- 2007 222 953

## Description

### TECHNICAL FIELD

The present invention relates to the field of electronic technologies, and in particular, to a projection apparatus and method.

### BACKGROUND

Nowadays, near-eye light field display is used in augmented reality (augmented reality, AR for short), virtual reality (virtual reality, VR for short) and other technologies, and can bring more real 3D display effects. Therefore, the near-eye light field display has become a development trend of future 3D display technologies. In a light field display technology, a wide-view-angle microprojection device is a basic component for generating a light field, and many manufacturers are investing much in research of the wide-view-angle microprojection device. In a digital light processing (Digital Light Processing, DLP) system, a size of a digital micromirror device (digital mirror device, DMD) determines a size of projection field of view (field of view, FOV), that is, a larger DMD indicates larger projection FOV, and a smaller DMD indicates smaller projection FOV. However, it is difficult to manufacture larger DMDs because of a current semiconductor manufacturing process. Projection FOV of a current DLP projection system is limited by a size of a DMD, and a corresponding projection angle of view of the projection FOV is too small to meet viewing requirements of users. In addition, large-size DMDs are expensive, and therefore, implementation costs for enlarging the projection FOV by increasing the size of the DMD are high.

In the prior art, a micro-electro-mechanical system (micro-electro-mechanical system, MEMS) is used to perform projection, and drives a mirror to perform scanning by using a piezoceramic motor or an electromagnetic technology, where the mirror rotates around a center. However, when the scanning is performed by the mirror rotating around the center, because projection FOV is limited by a maximum rotation angle of the mirror but currently the maximum rotation angle of the mirror is small, a projection angle of view corresponding to the projection FOV is still too small to meet viewing requirements of users.

In prior art, a fiber scanning and projection technology is used to perform projection. A micro motor drives an optical fiber to a resonance state, and fiber scanning is used to achieve a large projection angle of view to obtain a projection FOV that can meet viewing requirements of users. However, the prior art uses the micro motor to drive the optical fiber to the resonance state, which has high requirements on assembling. Factors including an extension length and material of the fiber and a position of the motor may all affect a resonance frequency of the entire system, and the operation is difficult and implementation costs are high.

From US 2007/0222953 A1 laser projection systems comprising an oscillating mirror are known. From WO 2007/116338 A2 scanning laser lighting devices comprising at least two laser light source for emitting light of different wavelengths are known. From CN 203 989 737 U projection display equipment is known. From JP 2014-085605 A projection systems with a movable mirror are known. From the US patent 5,727,098 display systems basing on an optical fiber with a movable second end are known. From WO 2015/129584 A1 projection apparatuses for projecting an image to be seen stereoscopically are known.

### SUMMARY

The invention is defined by the independent claims.

The dependent claims define advantageous embodiments.

### BRIEF DESCRIPTION OF DRAWINGS

To describe the technical solutions in the embodiments of the present invention more clearly, the following briefly describes the accompanying drawings required for describing the embodiments.
FIG. 1 is a schematic structural diagram of a projection apparatus according to an embodiment of the present invention;
FIG. 2 is another schematic structural diagram of a projection apparatus according to an embodiment of the present invention;
FIG. 3 is still another schematic structural diagram of a projection apparatus according to an embodiment of the present invention; and
FIG. 4 is a schematic flowchart of a projection method according to an embodiment of the present invention;

Reference Numerals:
1: Projection cavity; 11. Bottom of the projection cavity
12: Top of the projection cavity; 13: Inner wall of the projection cavity
3: Scanning motor; 31: Reflection layer
4: Processor; 41: Feedback unit
2: Light source; 5: Convex reflective surface
6: Curve corresponding to a projection angle of view 1; 61: Curve corresponding to a projection angle of view 2
7: Diffusion layer; 8: Photosensitive device

### DESCRIPTION OF EMBODIMENTS

The following clearly and completely describes the technical solutions in the embodiments of the present invention with reference to the accompanying drawings in the embodiments of the present invention. Apparently, the described embodiments are merely some but not all of the embodiments of the present invention. All other embodiments obtained by a person of ordinary skill in the art based on the embodiments of the present invention without creative efforts shall fall within the protection scope of the present invention as defined in the appended claims.

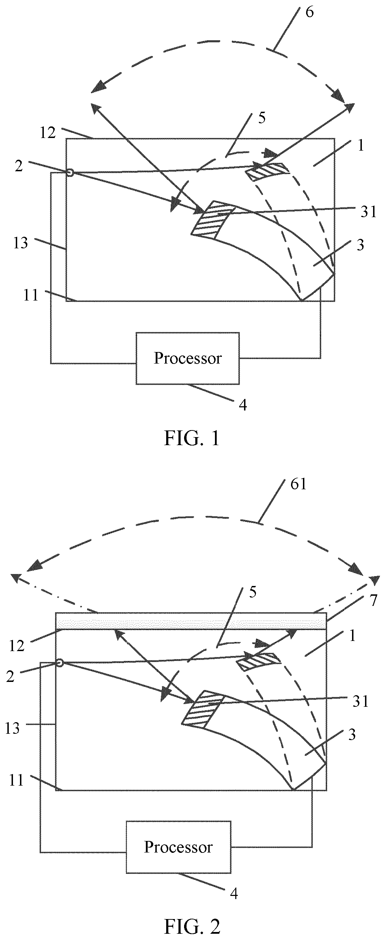

Referring to FIG. 1, FIG. 1 is a schematic structural diagram of a projection apparatus according to an embodiment of the present invention. The projection apparatus provided in the embodiment of the present invention includes a projection cavity 1, a light source 2, a scanning motor 3, and a processor 4.

In a specific implementation, the projection cavity 1 may be a polygonal prism projection cavity such as a cylindrical projection cavity, a cube projection cavity, a cuboid projection cavity, or a regular hexagonal prism projection cavity, which may be determined according to requirements of an actual application scenario or a manufacturing process of the apparatus and is not limited herein. The projection cavity shown in FIG. 1 is a front view of the projection cavity. The schematic diagram shown as FIG. 1 is used only for better describing the structural features of the projection apparatus, and more schematic drawings can be determined according to actual application scenarios. This is not limited herein.

In a specific implementation, the projection cavity may be formed by a bottom 11, a top 12 and an inner wall 13. If the projection cavity 1 is a cylindrical projection cavity, the inner wall 13 is a side surface of the projection cavity, and a stretch-out diagram of the side surface is a rectangle. If the projection cavity 1 is a cube projection cavity or a cuboid projection cavity, the inner wall 13 is four side surfaces of the projection cavity and each side surface is a square or a rectangle. If the projection cavity 1 is a regular hexagonal prism projection cavity, which is a polygonal prism projection cavity, the inner wall 13 is six side surfaces of the projection cavity and each side surface is a rectangle. The specific shape of the inner wall 13 of the projection cavity 1 may be determined according to an actual application scenario and is not limited herein.

The light source 2 and the scanning motor 3 are located inside the projection cavity 1. Specifically, the light source 2 is disposed on the inner wall 13 of the projection cavity, and the scanning motor 3 is disposed on a connecting edge of the inner wall 13 of the projection cavity and the bottom 11 of the projection cavity. Specifically, if the projection cavity 1 is a cylindrical projection cavity, the light source 2 is disposed at any position on the inner wall 13 of the projection cavity, and the scanning motor 3 may be disposed within an irradiation range of light emitted by the light source 2. If the projection cavity 1 is a polygonal prism projection cavity, the light source 2 may be disposed on any one of the side surfaces of the inner wall 13 of the projection cavity, and the scanning motor 3 may be disposed at a position on a surface opposite to the side surface on which the light source 2 is disposed, on a surface adjacent to a surface opposite to the side surface on which the light source 2 is disposed, or the like. The surface opposite to the side surface on which the light source 2 is disposed on or the surface opposite to the side surface on which the light source 2 is disposed and the adjacent surfaces thereof are any surface in the irradiation range of the light emitted by the light source 2. A fixing pin of the scanning motor 3 may be disposed on the inner wall 13 of the projection cavity 1, or on the bottom 11 of the projection cavity 1. The specific manner in which the scanning motor 3 is disposed may be determined according to an actual application scenario and is not limited herein. A reflection layer 31 is disposed on a scanning mirror of the scanning motor 3. The reflection layer 31 is configured to reflect the light emitted by the light source 2, to reflect the light emitted by the light source 2 outside the projection cavity 1. Specifically, a material of the reflection layer 31 may include an electroplated coating, a vapor deposition coating, or an adhesive reflector and the like. The electroplated coating may specifically be an electroplated metal layer, and the vapor deposition coating may specifically be a vapor deposition metal layer. The manner and material for fabricating the reflection layer 31 may specifically be determined according to an actual application scenario and are not limited herein.

Further, a shape of a cover area of the reflection layer 31 covering the scanning mirror may include a circle, an equilateral polygon or an irregular pattern and the like, which may specifically be determined according to the manufacturing process of the projection apparatus and is not limited herein. In embodiments of the present invention, the maximum diameter of the cover area of the reflection layer 31 equals the product of a linear velocity of rotation during rotary scanning of the scanning motor 3 and a pulse width of the light source, which can ensure the clarity of a projected image and improve the display effect of the projected image. If the cover area of the reflection layer 31 is a circle, the diameter of the circle is the maximum diameter of the cover area. If the cover area of the reflection layer 31 is an equilateral polygon, the straight line between a center of the equilateral polygon and each vertex is the maximum diameter. If the cover area of the reflection layer 31 is an irregular pattern, the irregular pattern may be divided into multiple small regular patterns, the maximum diameter of a small regular pattern among the small regular patterns is the maximum diameter of the cover area, and diameters of other small regular patterns are smaller than the maximum diameter of the cover area. The pulse width determines the resolution of the projected image formed by the reflected light. If the maximum diameter of the cover area of the reflection layer 31 is too large, the projected image becomes blurry. If the maximum diameter of the cover area of the reflection layer 31 is too small, pixels of the projected image are distributed discontinuously, affecting the display effect of the image.

Further, a shape of the scanning mirror covered by the reflection layer 31 is an arc surface, and an arc defined by the arc surface and a scanning arc defined by the scanning motor 3 during the rotary scanning are concentric arcs. When the scanning motor 3 performs the rotary scanning, a moving track of the reflection layer 31 on the scanning mirror of the scanning motor 3 can form a convex reflective surface. The convex reflective surface 5 as shown in FIG. 1 enables the scanning range of the light reflected outside the projection cavity to be larger than the scanning range of the scanning motor. The convex reflective surface 5 and the arc defined by the shape of the scanning mirror covered by the reflection layer 31 are concentric arcs. When the area of the reflection layer 31 is small enough, the shape of the scanning mirror covered by the reflection layer 31 is approximately a plane. When the area of the reflection layer is small enough, a central angle of the arc corresponding to the scanning mirror covered by the reflection layer is θ, which satisfies a condition that sinθ approximately equals θ.

The processor 4 may be disposed inside the projection cavity 1 or outside the projection cavity 1. This is not limited herein. The following description is provided by using an example in which the processor is disposed outside the projection cavity 1. The processor 4 is connected to both the light source 2 and the scanning motor 3.

In a specific implementation, the processor 4 can preset a scanning model for driving the scanning motor 3 to perform the rotary scanning, and store the scanning model in a memory (not shown in FIG. 1) of the projection apparatus. When the projection apparatus starts a working mode, the processor 4 can obtain, from the memory, a specified scanning mode output by the scanning model, and drive the scanning motor 3 to perform the rotary scanning according to the specified scanning mode. Specifically, the processor 4 can determine, according to the preset specified scanning mode, scanning parameters such as a rotary direction, a scanning frequency, and a required scanning voltage during the rotary scanning of the scanning motor, and further can drive the scanning motor to perform the rotary scanning according to the scanning parameters. The specified scanning mode may include a Z-scanning mode, a spiral scanning mode, or a circular scanning mode. This is not limited herein.

Further, in a specific implementation, the processor 4 can determine, according to a relative position relationship between the scanning mirror and the light source 2 during the rotary scanning of the scanning motor 3, a direction of incident light on the reflection layer 31 of the scanning mirror when the light emitted by the light source irradiates on the scanning mirror, and further determine a direction of reflected light when the incident light is reflected by the reflection layer 31. After the processor 4 determines the direction of the reflected light, light parameters of the light emitted by the light source can be determined according to data of an image to be projected stored in the memory, so that an image formed by the reflected light is the same as the image to be projected. The light parameters include light intensity, light colors and the like. After determining the light parameters, the processor 4 can drive the light source 2 according to the light parameters to generate light corresponding to the light parameters, and emit the light into the projection cavity, and the light is reflected outside the projection cavity 1 by means of the reflection layer 31 on the scanning mirror of the scanning motor.

For example, assuming that at a moment A, the scanning motor 3 reaches a position A1 (the solid line in FIG. 1) during the rotary scanning, the processor 4 can, according to the position A1 of the scanning motor 3 and the position of the light source 2, determine an angle of incidence α1 of the incident light that is the light emitted by the light source 2 and that is irradiated on the reflection layer 31 at the moment. Further, the processor 4 can determine, according to the angle of incidence α1, an angle of reflection of the reflected light generated after the incident light is reflected by the reflection layer 31, and can further determine, according to the data of the image to be projected, light parameters such as light intensity and light colors required by projection in the angle of reflection. Assuming that at a moment B, the scanning motor 3 reaches a position B1 (the dashed line in FIG. 1) during the rotary scanning, the processor 4 can determine, according to the position B1 of the scanning motor 3 and the position of the light source 2, an angle of incidence α2 of the incident light that is the light emitted by the light source 2 and that is irradiated on the reflection layer 31 at the moment. Further, the processor 4 can determine, according to the angle of incidence α2, an angle of reflection of the reflected light generated after the incident light is reflected by the reflection layer 31, and can further determine, according to the data of the image to be projected, light parameters such as light intensity and light colors required by projection in the angle of reflection.

Through the rotary scanning of the scanning motor 3 and the reflection of light by the reflection layer 31, the projection apparatus enables the scanning range of the light reflected outside the projection cavity to be larger than the scanning range of the scanning motor, thereby achieving a larger projection angle of view (which is assumed to a be projection angle of view 1), for example, a central angle (not shown in FIG. 1) corresponding to the curve 6. The central angle corresponding to the curve 6 can be determined according to the angle of reflection of the light reflected by the reflection layer of the scanning motor 3 at the moment A and the angle of reflection of the light reflected by the reflection layer of the scanning motor 3 at the moment B. The specific angle may be determined according to an actual application scenario, and further details are not described herein.

Referring to FIG. 2, FIG. 2 is another schematic structural diagram of a projection apparatus according to an embodiment of the present invention.

In some feasible implementations, the projection apparatus provided by this embodiment of the present invention further includes a diffusion layer 7. The diffusion layer 7 may include a lens layer or a grating layer and the like, which may be determined according to the actual manufacturing process, and is not limited herein. Further, parameters such as the thickness and size of the diffusion layer 7 may also be determined according to factors such as the requirements of an actual application scenario and the manufacturing process, and are not limited herein.

In a specific implementation, the diffusion layer 7 is disposed on a top 12 of the projection cavity, and is configured to diffuse light reflected by the reflection layer 31 outside the projection cavity to achieve a larger projection angle of view (which is assumed to be a projection angle of view 2), for example, a central angle (not shown in FIG. 2) corresponding to the curve 61. The central angle corresponding to the curve 61 can be determined according to the angle of reflection of the light reflected by the reflection layer 31 of the scanning motor 3 at the moment A, the angle of reflection of the light reflected by the reflection layer 31 of the scanning motor 3 at the moment B, and light diffusion parameters (for example, a refractive index of a lens) of the material of the diffusion layer 7. The specific angle may be determined according to an actual application scenario, and further details are not described herein.

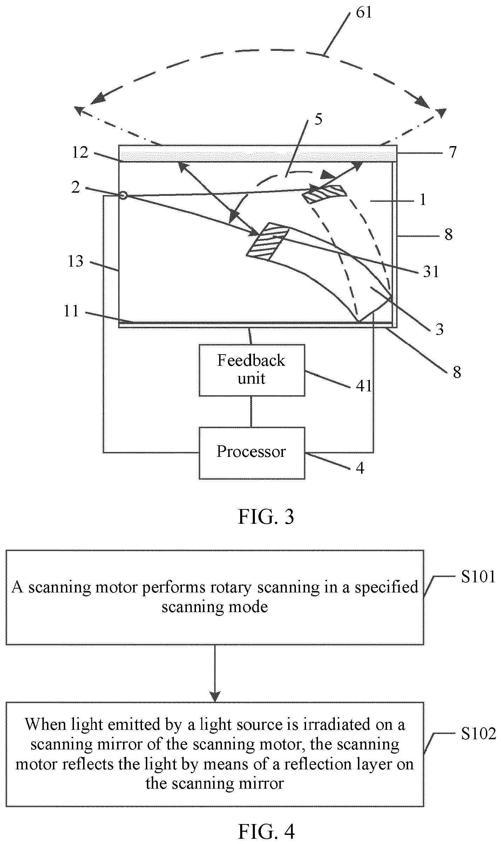

Referring to FIG. 3, FIG. 3 is still another schematic structural diagram of a projection apparatus according to an embodiment of the present invention.

In some feasible implementations, the projection apparatus provided by this embodiment of the present invention further includes a photosensitive device 8 and a feedback unit 41. The photosensitive device 8 is disposed on the inner wall 13 of the projection cavity 1 or on the bottom 11 of the projection cavity 1, or is disposed on the inner wall 13 and the bottom 11 of the projection cavity 1. If the projection cavity 1 is a cylindrical projection cavity, the photosensitive device 8 can be disposed on the inner wall with a specified area defined by a position of the fixing pin of the scanning motor 3. For example, assuming that the position of the fixing pin of the scanning motor 3 is M on the inner wall of the projection cavity 1, a parallel line L of the connecting edge of the inner wall 13 and the bottom 11 of the projection cavity 1 can be determined with M as a center. Assuming that the length of the parallel line L is a, and the height of the projection cavity 1 (that is, the width of the stretch-out diagram of the inner wall 13) is b, the area of a*b can be used as the specified area that is defined by the position of the fixing pin of the scanning motor 3. The photosensitive device 8 can be disposed on the inner wall with the specified area. If the projection cavity 1 is a polygonal prism projection cavity, the fixing pin of the scanning motor 3 is disposed on one of the multiple side surfaces of the inner wall 13. Assuming that the fixing pin of the scanning motor 3 is disposed on a side surface 1, the photosensitive device 8 can be disposed on the side surface 1. In a specific implementation, the position of the photosensitive device 8 on the inner wall or the bottom of the projection cavity 1 can be defined according to the position of the scanning motor 3. When light emitted by the light source 2 is irradiated on the scanning motor 3, the scanning mirror of the scanning motor 3 transmits the light of the light source 2, so that the scanning motor 3 forms a shadow on the bottom or the inner wall of the projection cavity 1. The photosensitive device 8 may be disposed at any position within the range of the shadow formed by the scanning motor 3 on the bottom or the inner wall of the projection cavity 1, for sensing the shadow of the scanning mirror of the scanning motor 3.

One end of the feedback unit 41 is connected to the photosensitive device 8, and the other end of the feedback unit 41 is connected to the processor 4. The feedback unit 41 is configured to calculate a position of the scanning mirror of the scanning motor 3 according to the shadow sensed by the photosensitive device 8, and feed back the position to the processor 4. The processor 4 compares a measured value of the position of the scanning mirror fed back by the feedback unit 41 with a measured value of the position of the scanning mirror estimated according to the preset scanning model, and determines a difference value between the measured values of the two positions, and can further correct the scanning model according to the difference value. The scanning model is corrected according to the feedback of the feedback unit 41, so the accuracy of estimating the position of the scanning mirror according to the scanning model is improved, and further, the quality of projected images and robustness of the system can be improved.

Referring to FIG. 4, FIG. 4 is a schematic flowchart of a projection method according to an embodiment of the present invention. The projection method provided by the embodiment of the present invention includes the following steps.
S101. A scanning motor performs rotary scanning in a specified scanning mode; and
S102. When light emitted by a light source is irradiated on a scanning mirror of the scanning motor, the scanning motor reflects the light by means of a reflection layer on the scanning mirror. In some feasible implementations, the specified scanning mode includes at least one of a Z-scanning mode, a spiral scanning mode, or a circular scanning mode.

In some feasible implementations, a shape of the scanning mirror covered by the reflection layer is an arc surface; and
an arc defined by the arc surface and a scanning arc defined by the scanning motor during the rotary scanning are concentric arcs.

In a specific implementation, for an implementation performed by the scanning motor in the projection method, refer to the implementation performed by the scanning motor in the foregoing embodiments, and details are not described herein again.

A person of ordinary skill in the art may understand that all or some of the processes of the methods in the embodiments may be implemented by a computer program instructing relevant hardware. The program may be stored in a computer readable storage medium. When the program runs, the processes of the methods in the embodiments are performed. The foregoing storage medium may include: a magnetic disk, an optical disc, a read-only memory (Read-Only Memory, ROM), or a random access memory (Random Access Memory, RAM).

## Claims

1. A projection apparatus, comprising a projection cavity (1), a light source (2), a scanning motor (3), and a processor (4), wherein
the processor (4) is connected to both the light source (2) and the scanning motor (3) and is configured to determine scanning parameters and light parameters; wherein a scanning mirror of the scanning motor is covered by a reflection layer (31), the reflection layer (31) being configured to reflect light emitted by the light source (2) outside of the projection cavity;
wherein the projection apparatus is a near-eye projection apparatus; and
wherein the light source (2) and the scanning motor (3) are located inside the projection cavity (1);
**characterized in that**
the scanning mirror covered by the reflection layer (31) is a surface with a first arc-shaped cross section, **in that**
the reflection layer (31) forms a convex reflective surface (5) when the scanning motor (3) performs scanning, the convex reflective surface (5) having a second arc-shaped cross-section, and **in that**
the first arc-shaped cross-section and the second arc-shaped cross-section are concentric,
such that a scanning range of the light reflected outside the projection cavity is larger than a scanning range of the scanning motor (3).

2. The projection apparatus according to claim 1, wherein the reflection layer (31) comprises at least one of an electroplated coating, a vapor deposition coating, or an adhesive reflector.

3. The projection apparatus according to any one of claims 1 to 2, wherein the projection cavity (1) is formed by a bottom (11), a top (12) and an inner wall (13), and
wherein a diffusion layer (7) is disposed on the top of the projection cavity (1), the diffusion layer (7) being configured to diffuse the light reflected by the reflection layer (31) outside the projection cavity (1).

4. The projection apparatus according to claim 3, wherein the diffusion layer (7) comprises a lens layer or a grating layer.

5. The projection apparatus according to any one of claims 1 to 2, wherein the projection cavity (1) is formed by a bottom (11), a top (12) and an inner wall (13),
wherein the light source (2) is located on the inner wall (13) of the projection cavity (1), and
wherein the scanning motor (3) is located on a connecting edge of the inner wall (13) of the projection cavity (1) and the bottom (11) of the projection cavity (1).

6. The projection apparatus according to claim 5, wherein the projection apparatus further comprises a photosensitive device (8) and a feedback unit (41), wherein
the photosensitive device (8) is located on the inner wall (13) of the projection cavity (1) or the bottom (11) of the projection cavity (1), and
wherein one end of the feedback unit (41) is connected to the photosensitive device (8), and the other end of the feedback unit (41) is connected to the processor (4).

7. A near-eye projection method, to be performed by a projection apparatus according to one of claims 1 to 6, the method comprising:
performing, by the scanning motor (3), scanning in a specified scanning mode (S101); and
when light emitted by the light source (2) is irradiated on the scanning mirror of the scanning motor (3), reflecting, by the scanning motor (3), the light by means of the reflection layer (31) on the scanning mirror (S102).

8. The projection method according to claim 7, wherein the specified scanning mode comprises at least one of a Z-scanning mode, a spiral scanning mode, or a circular scanning mode.

## Patentansprüche

1. Projektionsvorrichtung, umfassend einen Projektionshohlraum (1), eine Lichtquelle (2), einen Abtastmotor (3) und einen Prozessor (4), wobei
der Prozessor (4) mit sowohl der Lichtquelle (2) als auch dem Abtastmotor (3) verbunden ist und ausgestaltet ist, um Abtastparameter und Lichtparameter zu bestimmen; wobei ein Abtastspiegel des Abtastmotors mit einer Reflexionsschicht (31) bedeckt ist, die Reflexionsschicht (31) ausgestaltet ist, um durch die Lichtquelle (2) emittiertes Licht an die Außenseite des Projektionshohlraums zu reflektieren;
wobei die Projektionsvorrichtung eine augennahe Projektionsvorrichtung ist; und
wobei die Lichtquelle (2) und der Abtastmotor (3) sich innerhalb des Projektionshohlraums (1) befinden;
**dadurch gekennzeichnet, dass**
der mit der Reflexionsschicht (31) bedeckte Abtastspiegel eine Oberfläche mit einem ersten bogenförmigen Querschnitt ist, die Reflexionsschicht (31) eine konvexe reflektierende Oberfläche (5) bildet, wenn der Abtastmotor (3) das Abtasten durchführt, die konvexe reflektierende Oberfläche (5) einen zweiten bogenförmigen Querschnitt aufweist, und der erste bogenförmige Querschnitt und der zweite bogenförmige Querschnitt konzentrisch sind, so dass ein Abtastbereich des an die Außenseite des Projektionshohlraums reflektierten Lichts größer als ein Abtastbereich des Abtastmotors (3) ist.

2. Projektionsvorrichtung nach Anspruch 1, wobei die Reflexionsschicht (31) mindestens eins von einer elektroplattierten Beschichtung, einer Dampfphasenabscheidungsbeschichtung oder einem Klebereflektor umfasst.

3. Projektionsvorrichtung nach einem der Ansprüche 1 bis 2, wobei der Projektionshohlraum (1) aus einem Boden (11), einem Oberteil (12) und einer Innenwand (13) gebildet ist, und
wobei eine Streuschicht (7) oben auf dem Projektionshohlraum (1) angeordnet ist, wobei die Streuschicht (7) ausgestaltet ist, um das durch die Reflexionsschicht (31) reflektierte Licht an die Außenseite des Projektionshohlraums (1) zu streuen.

4. Projektionsvorrichtung nach Anspruch 3, wobei die Streuschicht (7) eine Linsenschicht oder eine Beugungsgitterschicht umfasst.

5. Projektionsvorrichtung nach einem der Ansprüche 1 bis 2, wobei der Projektionshohlraum (1) aus einem Boden (11), einem Oberteil (12) und einer Innenwand (13) gebildet ist, die Lichtquelle (2) sich auf der Innenwand (13) des Projektionshohlraums (1) befindet, und wobei der Abtastmotor (3) sich auf einer Verbindungskante der Innenwand (13) des Projektionshohlraums (1) und des Bodens (11) des Projektionshohlraums (1) befindet.

6. Projektionsvorrichtung nach Anspruch 5, wobei die Projektionsvorrichtung des Weiteren eine lichtempfindliche Einrichtung (8) und eine Feedback-Einheit (41) umfasst, wobei die lichtempfindliche Einrichtung (8) sich auf der Innenwand (13) des Projektionshohlraums (1) oder dem Boden (11) des Projektionshohlraums (1) befindet, und
wobei ein Ende der Feedback-Einheit (41) mit der lichtempfindlichen Einrichtung (8) verbunden ist und das andere Ende der Feedback-Einheit (41) mit dem Prozessor (4) verbunden ist.

7. Augennahes Projektionsverfahren, das durch eine Projektionsvorrichtung nach einem der Ansprüche 1 bis 6 durchzuführen ist, wobei das Verfahren umfasst:
Durchführen von Abtasten in einem spezifizierten Abtastmodus durch den Abtastmotor (3) (S101); und
wenn durch die Lichtquelle (2) emittiertes Licht auf den Abtastspiegel des Abtastmotors (3) gestrahlt wird, Reflektieren des Lichts durch den Abtastmotor (3) mittels der Reflexionsschicht (31) auf dem Abtastspiegel (S102).

8. Projektionsverfahren nach Anspruch 7, wobei der spezifizierte Abtastmodus mindestens einen von einem Z-Abtastmodus, einem Spiralabtastmodus oder einem kreisförmigen Abtastmodus umfasst.

## Revendications

1. Appareil de projection, comprenant une cavité de projection (1), une source de lumière (2), un moteur de balayage (3), et un processeur (4),
le processeur (4) étant connecté à la fois à la source de lumière (2) et au moteur de balayage (3) et étant configuré pour déterminer des paramètres de balayage et des paramètres de lumière ;
un miroir de balayage du moteur de balayage étant couvert par une couche de réflexion (31), la couche de réflexion (31) étant configurée pour réfléchir la lumière émise par la source de lumière (2) à l'extérieur de la cavité de projection ;
l'appareil de projection étant un appareil de projection proche de l'œil ; et
la source de lumière (2) et le moteur de balayage (3) étant situés à l'intérieur de la cavité de projection (1) ;
**caractérisé en ce que**
le miroir de balayage recouvert par la couche de réflexion (31) est une surface ayant une première section transversale en forme d'arc, **en ce que** la couche de réflexion (31) forme une surface réfléchissante convexe (5) lorsque le moteur de balayage (3) effectue un balayage, la surface réfléchissante convexe (5) ayant une seconde section transversale en forme d'arc, et **en ce que** la première section transversale en forme d'arc et la seconde section transversale en forme d'arc sont concentriques, de sorte qu'une plage de balayage de la lumière réfléchie à l'extérieur de la cavité de projection est plus grande qu'une plage de balayage du moteur de balayage (3).

2. Appareil de projection selon la revendication 1, la couche de réflexion (31) comprenant au moins l'un d'un revêtement électrodéposé, d'un revêtement par dépôt en phase vapeur ou d'un réflecteur adhésif.

3. Appareil de projection selon l'une quelconque des revendications 1 et 2, la cavité de projection (1) étant formée par un fond (11), un sommet (12) et une paroi interne (13), et une couche de diffusion (7) étant disposée sur le sommet de la cavité de projection (1), la couche de diffusion (7) étant configurée pour diffuser la lumière réfléchie par la couche de réflexion (31) à l'extérieur de la cavité de projection (1).

4. Appareil de projection selon la revendication 3, la couche de diffusion (7) comprenant une couche de lentille ou une couche de réseau.

5. Appareil de projection selon l'une quelconque des revendications 1 et 2, la cavité de projection (1) étant formée par un fond (11), un sommet (12) et une paroi interne (13), la source de lumière (2) étant située sur la paroi interne (13) de la cavité de projection (1), et le moteur de balayage (3) étant situé sur un bord de connexion de la paroi interne (13) de la cavité de projection (1) et du fond (11) de la cavité de projection (1).

6. Appareil de projection selon la revendication 5,
l'appareil de projection comprenant en outre un dispositif photosensible (8) et une unité de rétroaction (41), le dispositif photosensible (8) étant situé sur la paroi interne (13) de la cavité de projection (1) ou le fond (11) de la cavité de projection (1), et une extrémité de l'unité de rétroaction (41) étant connectée au dispositif photosensible (8), et l'autre extrémité de l'unité de rétroaction (41) étant connectée au processeur (4).

7. Procédé de projection à proximité de l'œil, à réaliser par un appareil de projection selon l'une des revendications 1 à 6, le procédé comprenant :
la réalisation, par le moteur de balayage (3), d'un balayage dans un mode de balayage spécifié (S101) ; et
lorsque la lumière émise par la source de lumière (2) est irradiée sur le miroir de balayage du moteur de balayage (3), la réflexion, par le moteur de balayage (3), de la lumière au moyen de la couche de réflexion (31) sur le miroir de balayage (S 102).

8. Procédé de projection selon la revendication 7, le mode de balayage spécifié comprenant au moins l'un d'un mode de balayage en Z, d'un mode de balayage en spirale, ou d'un mode de balayage circulaire.
